# EUROPEAN PATENT APPLICATION

(11) **EP 2 600 148 A1**
(43) Date of publication of application: **05.06.2013**
(21) Application number: 11009445.5
(22) Date of filing: 29.11.2011
(51) Int. Cl.: G01N 33/487

(54) **Device and method for monitoring transport activity across a cell membrane**

(71) Applicant: ETH Zurich, 8092 Zurich (CH)
(72) Inventor: Schaffhauser, Daniel, 8051 Zürich (CH); Dittrich, Petra, 8105 Watt (CH)

(57) **Abstract**

In a microfluidic device (1) for monitoring transport activity across a cell membrane (3) of a cell (2), the device (1) comprising a cell chamber (11) and a perfusion channel (12), wherein the cell chamber (11) is connected with the perfusion channel (12) through an opening (14), it is according to the invention proposed that the microfluidic device (1) further comprises a sensing element (13) having a sensing surface (15), wherein the sensing surface (15) is arranged in the perfusion channel (12) and aligned with the opening (14) such that the sensing surface (15) is completely coverable by a cell membrane (3) of a cell (2) placed in the cell chamber (11). In addition a method is proposed for monitoring transport activity across a cell membrane by establishing a proton-selective diffusion barrier between the sensing surface (15) and the perfusion channel (12).

## Description

### TECHNICAL FIELD

The invention is directed to a microfluidic device for monitoring transport activity across a cell membrane of a cell, the device comprising a cell chamber and a perfusion channel, wherein the cell chamber is connected with the perfusion channel through an opening. The invention is further directed to a method for monitoring transport activity across a cell membrane of a cell.

### PRIOR ART

For many decades, electrophysiological methods have been at the forefront of investigations of cell membrane conduction and excitation in living cells. To measure cell membrane conductance, control of either the transmembrane voltage or current is required. Therefore, a key experimental challenge is to establish adequate electrical access to the cytosol. The most common measuring techniques are the Two-Electrode Voltage Clamp (TEVC) technique for large cells (e.g. *Xenopus laevis* oocytes) and the Patch Clamp technique for small cells.

For example, in the commonly used two electrode voltage clamp (TEVC) applied to large cells such as *Xenopus laevis* oocytes, two microelectrodes impale the oocyte to sense and control the membrane potential. This procedure requires delicate glass microelectrodes and a high degree of micromanipulation, either by a human operator or precision robotics.

In the whole cell patch clamp, electrical access to the cytosol is gained by sealing the tip of a glass microelectrode to the membrane and applying suction to eventually rupture the cell membrane within the electrode.

Recently, several non-invasive voltage clamp techniques for *Xenopus laevis* oocytes have been developed that leave the membrane intact. These methods rely on the physical compartmentalization of the membrane into two areas and measurement of trans-cellular currents across the entire oocyte. In the transoocyte voltage clamp (TOVC) an AC voltage is applied across symmetrically distributed membrane impedances. (D. Cucu, J. Simaels, D. Jans, W. Van Driessche, 2004, Pflugers Arch447, 934)

Dahan E et al. (Dahan E et al., Rapid fluidic exchange microsystem for recording of fast ion channel kinetics in Xenopus oocytes, Lab Chip, 2008, 8, 1809-18) describes a device for the electrophysiological measurement of a membrane potential on oocytes, wherein the measurement is based on the TEVC-method. The device comprises a microfluidic chip with a conical shaped well as a cell compartment, which is open to the top side of the chip, and a perfusion channel underneath the cell compartment. The cell compartment and the perfusion channel are connected to each other by a tiny hole, which forms the measuring site where the cell has to be placed, similar to the known planar patch clamp technique. The oocyte is sucked and immobilized onto the hole by a stable negative pressure gradient. The pressure gradient is produced by pneumatic control system. The device of Dahan has the disadvantage that the pneumatic control system needs a lot of space making the miniaturization that is needed for high-throughput screening on e.g. 96- or 384-well plates impossible. Another disadvantage is the fact that the leak around the cell depends on the pressure differentials in the perfusion system.

Schaffhauser DF et al. (Schaffhauser DF et al., Microfluidic platform for electrophysiological studies on Xenopus laevis oocytes under varying gravity levels, 2011, Lab Chip, 11(20):3471-8) describes a mulitlayered microfluidic device with a cell chamber and a perfusion channel connected by a tiny hole. The mulitlayered microfluidic device provides an immobilization site for *Xenopus laevis* oocytes on an intermediate layer, and fluid and electrical connections from either side of the cell. A constant low level air pressure is applied to the oocyte to ensure a stable immobilization, which is essential for keeping the leak resistance constant. With the device the impedances of the two membrane areas are distributed asymmetrically, so that most of the voltage drop will occur across the patched membrane area. The device also permits electrical access to the cytosol of the oocyte without physical introduction of electrodes by e.g. permeabilisation of a large region of the oocyte membrane so that a defined membrane patch can be voltage clamped.

Even though voltage clamping has established itself as a reliable tool for performing electrophysiological experiments, its application in large-scale industrial screening has not been fully realized. Apart from the aforementioned cell manipulation requirements, parallelization of the voltage clamp requires duplication of many hardware components - including the electrodes, voltage clamp electronics and the fluidic pathways. Also, because in voltage clamping the current across the cell membrane is recorded, multichannel systems require the fluidic domains on both sides of the membrane to be completely isolated from each other. These issues complicate their implementation in high-throughput/multichannel electrophysiology systems. Nonetheless, automated commercial Voltage Clamp systems have been realized such as the OpusXpress 6000A (Axon Instruments) or the Port-a-Patch (Nanion Technologies). In the former, a 6-channel robotic TEVC is offered which utilizes motorized micromanipulators in order to gain electrical access to the cytosol. The OpusXpress 6000A is designed for the use of *Xenopus* oocytes. A disadvantage of the OpusXpress 6000A is a very large size of the channels, a high cost per channel and a high liquid consumption. The Port-a-Patch is an automated voltage clamp tool on mammalian single cells. It is designed to be an easy-to-use table-top device for high fidelity recordings. The Port-a-patch is a single-channel device making it unsuitable for high-throughput screenings.

Electronic detection methods based on field-effect devices, on the other hand, are attractive alternatives to the above mentioned methods. Sensors have been developed for recording cell reactions of viable cells that are based on ion-sensitive field-effect transistors (ISFET). The ISFET, which is derived from the metal-oxide field-effect transistor (MOSFET), replaces the metal gate electrode on the insulator with the bulk solution, the potential of which is defined using an ion-sensitive reference electrode. Charge separation at the insulator-liquid interface then results in a detectable potential difference due to the double-layer capacitance between the reference electrode and the semiconducting channel. ISFETs have mostly been used to measure local changes in pH, such as acidification in cell cultures. An example of such an ISFET-sensor with source, drain and gate is known from WO 2009/144304. The sensor comprises a biocompatible layer having a heterostructure of group-III-nitrides. For the functionality of the sensor it is essential that an additional layer of adherent cells is attached to the biocompatible layer.

Sakata et al. (Sakata T, Miyahara Y, Noninvasive Monitoring of Transporter-Substrate Interaction at Cell Membrane, Anal.Chem.2008, 80, 1493-96) describes a method for detection of extracellular potential changes induced as a result of the interaction between transporting proteins and substrate at the cell membrane of oocytes using a field-effect transistor (FET). The potential change at the cell membrane/gate insulator interface can be monitored during the uptake of substrate mediated by transporter protein. The device of Sakata et al. is based on a FET with a Si₃N₄ insulator layer. A cell compartment including a reference electrode is provided around the sensing area of the FET. During measurements oocytes are placed in the cell compartment and allowed to settle down on the sensing area by gravitational force. The disadvantage of the device is the unstable immobilization of the cell on the sensor. Also substances to be tested have to be added to the cell compartment and the changing of substances require washing steps. Every change in liquid surrounding the oocyte, e.g. when adding substances to be tested in screening, risks to move the oocyte away from gate area and impair the measurement.

### EXPLANATION OF THE INVENTION

It is an objective to provide a reliable device and a method for monitoring transport activities across the cell membrane of a cell. The device should be easy to use and applicable in high-throughput screening at a low-per-channel cost.

This is achieved with a microfluidic device according to claim 1 and the method according to claim 11. In particular, the microfluidic device according to the invention for monitoring transport activity across a cell membrane of a cell comprises a cell chamber and a perfusion channel, wherein the cell chamber is connected with the perfusion channel through an opening. The microfluidic device further comprises a sensing element having a sensing surface , wherein the sensing surface is arranged in the perfusion channel and aligned with the opening such that the sensing surface is completely coverable by a cell membrane of a cell placed in the cell chamber e.g. when partly squeezed through the opening.

The method according to the invention for monitoring transport activity across a cell membrane of a cell comprises the steps of (i) providing a cell in the cell chamber of the microfluidic device according to the invention, (ii) establishing a proton-selective diffusion barrier between the sensing surface and the perfusion channel, and (iii) monitoring a relative change in proton concentration at the sensing surface due to transport activitiy across the cell membrane of the cell.

*Xenopus laevis* oocytes, like other cells, are believed to have endogenous proton-regulating mechanisms at the cell membrane that are responsible for a pH gradient across the cell membrane. Furthermore, over expression of proton-dependent membrane proteins modulate local proton binding affinity upon transport activation. Considering the extremely fast bulk diffusion rate of protons in water, it may at first seem surprising that pH values different from the bulk solution can be detected at all. However, proton sinks and sources at the cell membrane change the local association/dissociation rate constants that results in lateral diffusion rates along the cell membrane different from the bulk diffusion rate. Recently, a mathematical model for lateral diffusion kinetics at the cell membrane for protons was described, which provides numerical solutions for the dwell time of a proton in buffered solutions commonly used in physiology (Medvedev ES, Stuchebrukhov AA, 2011, J Phys Condens Matter 23, 234103). The results support previously conducted experiments that show that a proton can migrate laterally hundreds of micrometers along the cell membrane before diffusing back into the bulk solution.

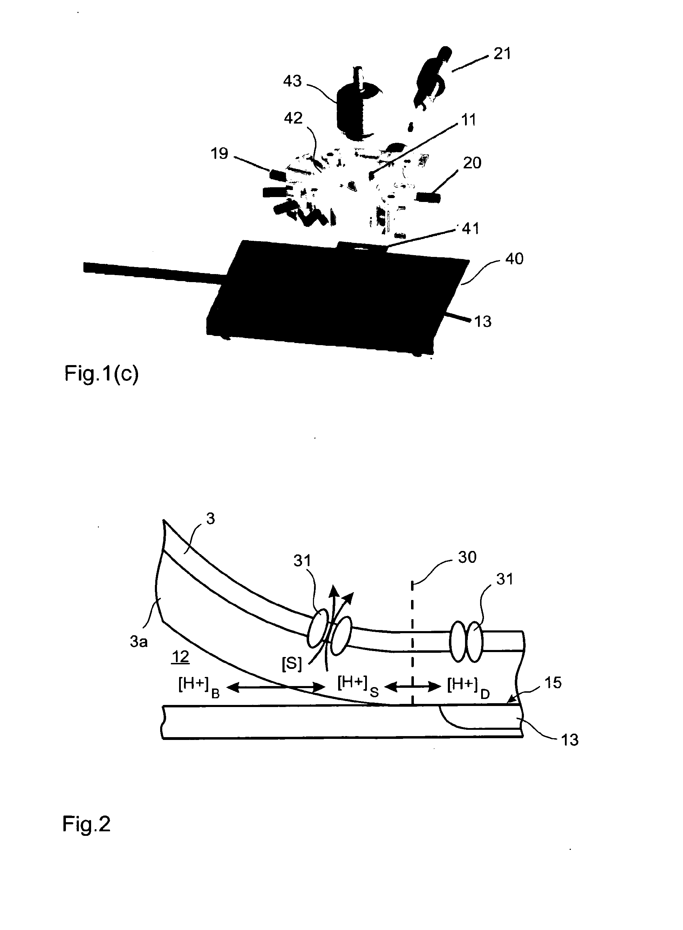

The microfluidic device according to the invention for monitoring proton-dependent membrane transport relies on local charge detection at the cell membrane due to lateral diffusion kinetics. With the device a proton-selective diffusion barrier can be established that is created by direct contact of part of the cell membrane with the sensing surface. Due to the much higher lateral diffusion rate of the protons compared to other solutes, the detected potential change at the sensing surface is exclusively proton-dependent (see Fig. 2 and detailed description further below). Also is the diffusion mechanism of protons vastly different from other ions. They behave like electrons where they act as a conjugated chain, whereas the diffusion of other ions is based on the individual particle.

The microfluidic devise according to the invention is easy to use because of the absence of the need for direct electrical access to the cytosol, no delicate preparative procedures like the impalement by microelectrodes (TEVC) or disruption by suction (Patch Clamp) are needed. Furthermore, it enables measurements on non-adherent cells. The device can be produced with an array of sensing elements and fluid supply channels, enabling the supply of chemical gradients to the cell array for systematic studies, which makes the device and the method suitable for multi-channel operation at low-per-channel cost and minimal space requirements, essentially opening the door to high-throughput, high quality single-cell electrophysiology. The method has the further advantage that electroneutral transport processes across the membrane can be monitored, which is not possible with clamping techniques.

Preferred embodiments of the invention are given in the dependent claims.

Preferably, the sensing surface as seen from the cell chamber through the opening into the perfusion channel is aligned with the center of the opening but lies on a plane parallel to the plane of the opening. The sensing surface forms part of a bottom side of the perfusion channel just below the opening (referring to the cell chamber as being above the opening), such that a cell partly squeezed through the opening can completely cover the sensing surface of the sensing element.

In a preferred embodiment the sensing element is a field-effect transistor (FET) with a source, a drain and a gate, preferably an ion-sensitive field-effect transistor (ISFET), and wherein the gate insulator material forms the sensing surface. Instead of a metalized gate, as usually used in the electronics industry, the metallization is replaced by the cell membrane and the physiological solution whose potential is defined by a reference electrode (usually 0V). Charge separation happens at the surface of the gate insulator surface (sensing surface) and is modulated by changes in local pH at the cell membrane. With such a FET or ISFET local charges at the cell membrane can be detected. The sensing surface can be formed by a pH-sensitive layer preferably of Si₃N₄, Al₂O₃ or Ta₂O₅.

In a further preferred embodiment of the invention the microfluidic device comprises immobilization means to immobilize the cell on the sensing surface. The immobilization means can be means for applying a pressure gradient force from the cell chamber through the opening towards the perfusion channel e.g. by applying an underpressure at an outlet of the perfusion channel to suck the cell into the opening and onto the sensing surface or by applying air overpressure in the cell chamber to press the cell onto the sensing surface. The immobilization means immobilize or fix the cell on the sensing surface, e.g. by partly squeezing the cell through the opening, and thereby maintain an appropriate coverage of the sensing surface by the cell membrane to establish the proton-selective diffusion barrier. The cell immobilization means also enable measurements on non-adherent cells. Furthermore, the immobilization of the cell creates three fluid compartments, namely the cell chamber, the perfusion channel and the cell itself. The fluid domains in the cell chamber and the perfusion channel can be exchanged separately form each other to allow supply of reagents, e.g. ion channel blocking compounds.

In a further preferred embodiment of the invention the perfusion channel comprises at least one inlet channel and one outlet channel. A perfusion system with several inlet channels connected to the perfusion channel has the advantage of easy and fast switching between different perfusion fluids containing different compounds to be tested.

In a further preferred embodiment of the invention a reference electrode is placed in the perfusion channel, preferably in the region of an outlet channel of the perfusion channel or in the outlet channel itself. By placing the electrode at the outlet an agar bridge is not necessary as dissociation products from the electrode are directly washed away, which greatly facilitates device integration.

In a further preferred embodiment of the mircofluidic device of the invention the source-drain pathway is held at a constant potential with constant current. The liquid domain above the gate insulator is kept at a constant potential via a reference electrode, e.g. a sintered Ag/AgCl pellet, against which the output signal, the potential difference between gate and source or gate and drain, is measured. This operating mode allows the direct readout of the potential difference between the bulk liquid and the gate insulator. The charge density at the drain island is modulated by the electrical field created by the polarization of the cell membrane or changes in local proton concentration.

The reference electrode can be printed on circuit board including the FET sensor element, which allows for larger and more cost-effective arrays of sensing elements for example for high-throughput screening devices. Also multiplexing between the several sensing elements can be done which greatly facilitates multi-channel operation and readout.

Another aspect of the invention is a multichannel microfluidic device for monitoring transport activity across a cell membrane of a cell comprising several microfluidic devices as described above, which are arranged in an array. Such a multichannel device allows for high-throughput screening with low cost-per-channel.

A further aspect of the invention is a method for monitoring transport activity across a cell membrane of a cell using the microfluidic device of the invention. The method comprises the following steps: (a) providing a cell in the cell chamber; (b) establishing a proton-selective diffusion barrier between the sensing surface and the perfusion channel; (c) monitoring a change in proton concentration at the sensing surface due to transport activity across the cell membrane of the cell. The method of the invention relies on the principle of local charge detection at the cell membrane by establishing the proton-selective diffusion barrier between the sensing surface and the perfusion channel.

In a preferred embodiment of the invention the proton-selective diffusion barrier is established by immobilizing and aligning the cell on the sensing surface, which is the active area of the sensing element. This can be achieved by applying a pressure gradient force from the cell chamber towards the perfusion channel, e.g. by applying air pressure in the cell chamber, to press the cell into the opening and onto the sensing surface or by sucking the cell into the opening and onto the sensing surface by application of negative pressure at the outlet of the perfusion system.

### BRIEF EXPLANATION OF THE FIGURES

The invention is described in greater detail below with reference to embodiments that are illustrated in the figures. The figures show:

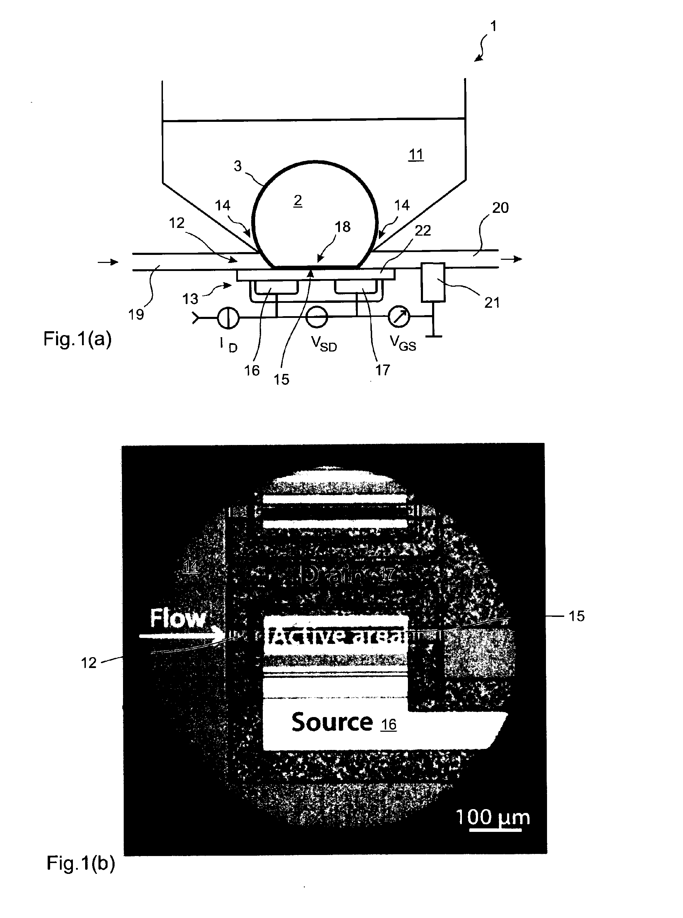

- Fig. 1: (a) a schematic drawing of a microfluidic device according to the invention; (b) a micrograph of the sensing surface as seen through the opening of the cell chamber; (c) exploded view of a microfluidic device according to the invention;
- Fig. 2: lateral diffusion model in a schematic close-up of the sensing surface of the device of Fig. 1 covered by a cell membrane;

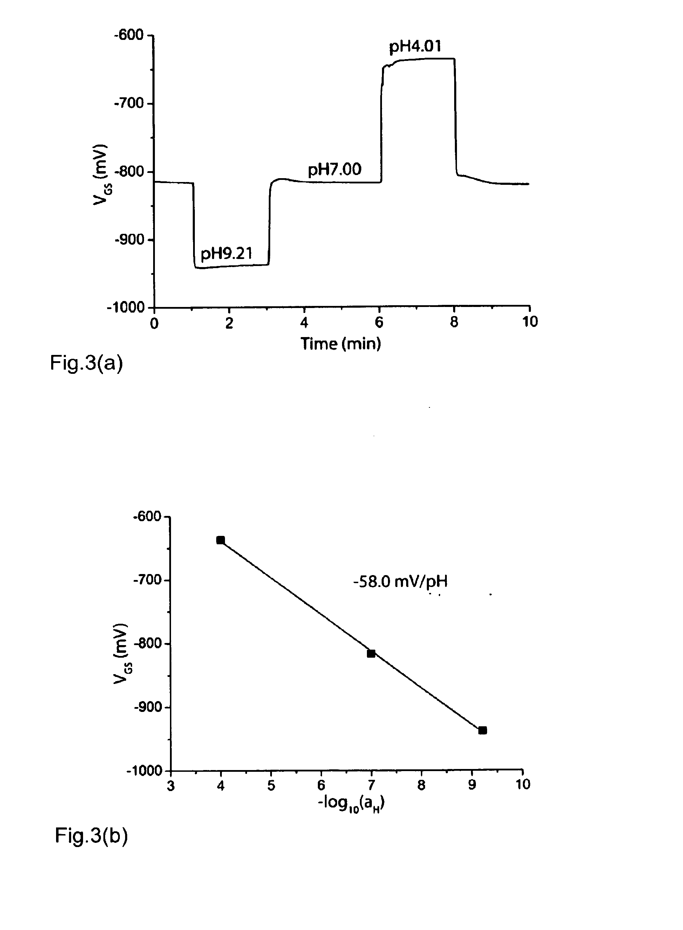

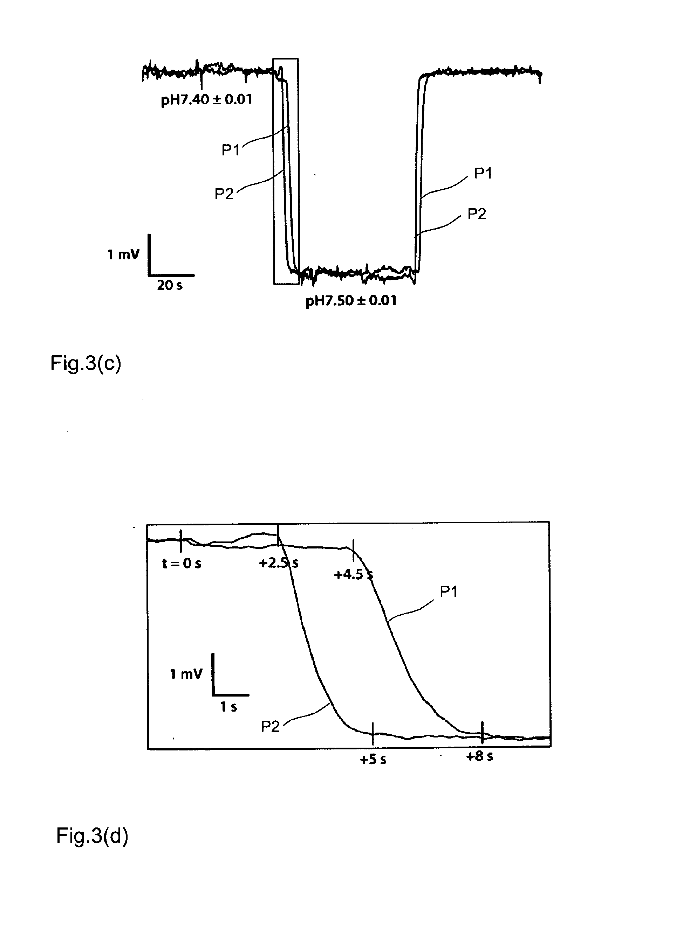

- Fig. 3: (a) Time-dependent measurement of phosphate reference buffers at pH 4.01, pH 7.00 and pH 9.21; (b) linear fit of the data points ob- tained from the baselines of each buffer; (c) Time-dependent measurement of 100 Na solutions buffered at pH 7.40 and pH 7.50 for two different perfusion pressures (100 mbar (P1) and 200 mbar (P2)); (d) Expanded view of data in panel (c) during the exchange of solutions from pH 7.40 to pH 7.50 (Valves were switched at t = 0s);

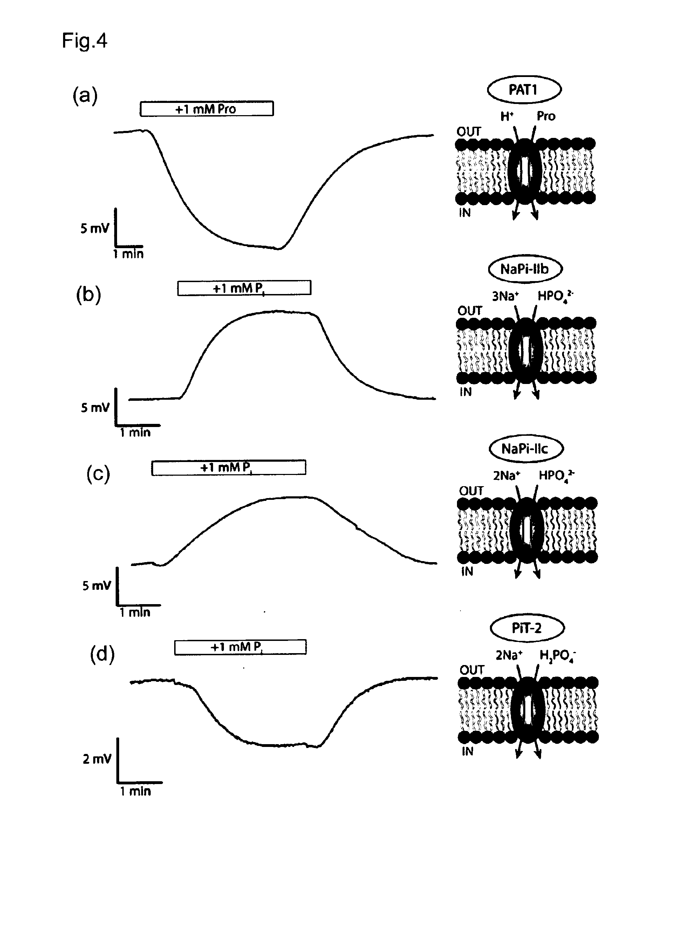

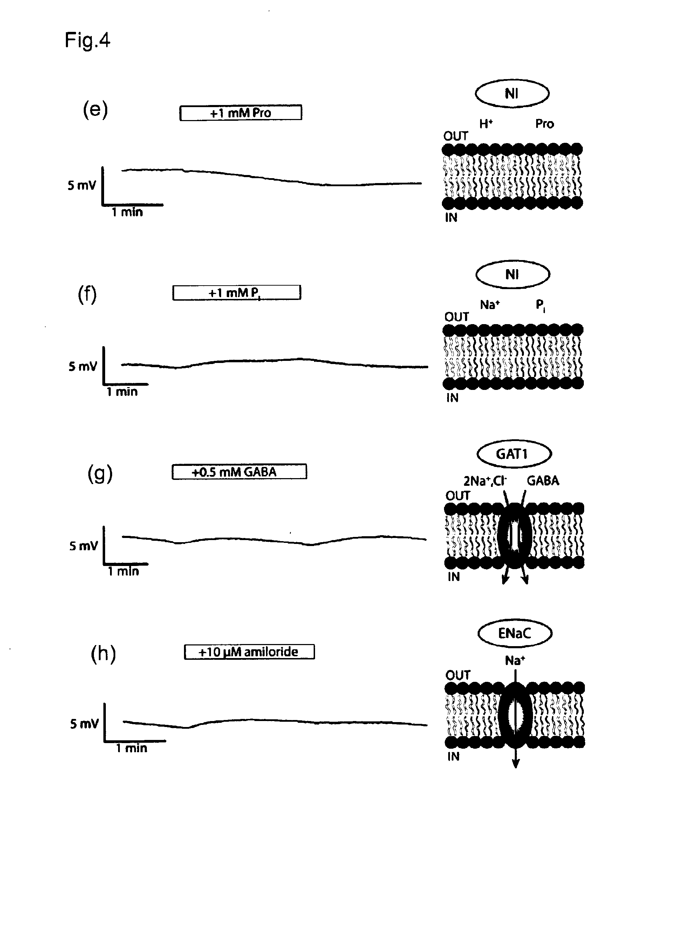

- Fig. 4: Experiments conducted on oocytes heterologously expressing various membrane transport proteins indicated with their respective controls on non-injected (NI) oocytes showing sensor readout (V_{GS}) as a function of time. Only part of the initial stabilizing baseline region that preceded substrate application is shown: (a) PAT1, (b) NaPi-IIb, (c) NaPi-IIc, (d) PiT-2, (e) Proline control, (f) Pi control, (g) GAT1, (h) ENaC; and

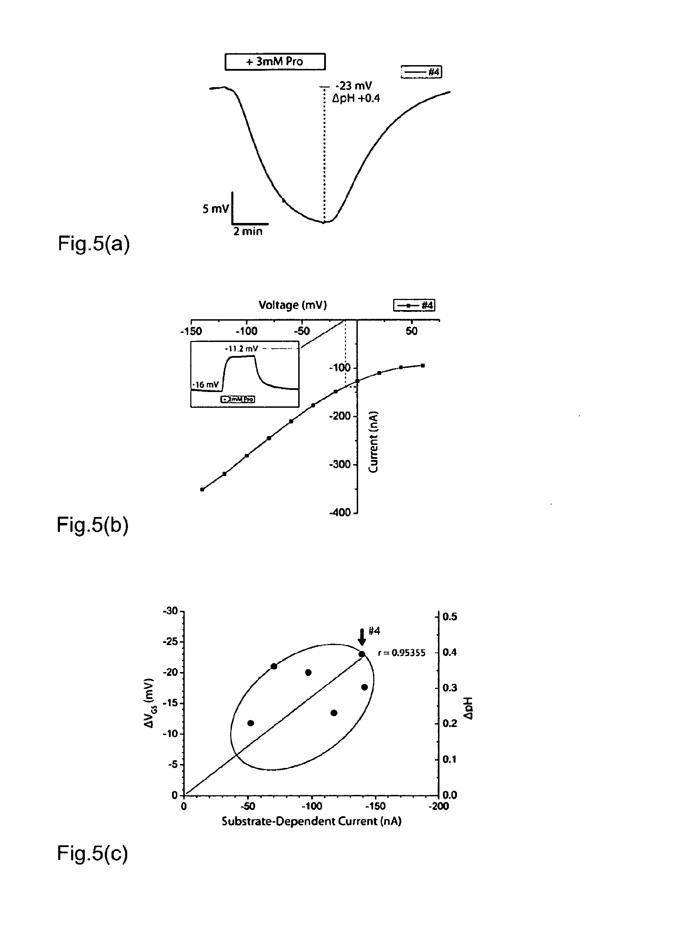

- Fig. 5: Correlation of sensor response with transport activity; (a) Sensor response to proline superfusion of a representative oocyte heterologously expressing PAT1; (b) TEVC I-V data of the proline-dependent current in response to the addition of 3 mM proline solution to the 100 Na buffer (Inset shows the change in membrane potential induced by proline application for the same oocyte as in (a)); (c) Correlation of V_{GS} and the substrate-dependent current (Each point represents data from a single oocyte).

### EMBODIMENTS OF THE INVENTION

Fig. 1(a) shows a schematic drawing of a microfluidic device 1 according to the invention for monitoring transport activities across the cell membrane 3 of a cell 2. The device comprises a cell chamber 11, a microperfusion system with a perfusion channel 12 and a sensing element 13. The arrows depict the liquid flow through the perfusion system. A circular opening 14 is provided between the cell chamber 11 and the perfusion channel 12 for immobilization of the cell 2 and alignment of the cell 2 with a sensing surface 15 of the sensing element 13. The sensing element 13 is placed in the perfusion channel 12 near the opening 14 such that the sensing surface 15 of the sensing element 13 can be completely covered by the cell membrane 3 of the cell 2. The height of the microperfusion channel 12 (or channel) is around 200 µm. A typical *Xenopus* oocyte would be 1000-2000 µm in diameter.

The sensing element 13 is a p-type or n-type field-effect transistor (FET) with source 16 and drain 17, with tantalum pentoxide (Ta₂O₅) as gate insulator material 22 and without metal gate. The sensing surface 15 is the area of the insulator material 22 between the source 16 and the drain 17. The gate 18 of the FET is formed by the cell membrane 3 of the cell 2 on the sensing surface 15.

As shown in Fig. 1(c) the sensing element 13 is inserted into a cavity of a precisely machined support 40 made of e.g. aluminum, creating a flush fit. A rectangular (approx. 5 mm x 10 mm) piece 41 of perfluoroethylene (PTFE) with a thickness of about 0.2 mm is placed over the sensing surface 15 to serve as a gasket and spacer and for forming the perfusion channel 12 of the microperfusion system. A CNC-machined block 42 made from poly(methyl methacrylate) (PMMA) is fixed onto the support 40. The block 42 comprises at least one liquid inlet channel 19 and an outlet channel 20 of the microperfusion system as well as the cell chamber 11 and the opening 14 for immobilization of the cell 2. An opening (about 0.8 to 1.6 mm dia.) intersecting with the outlet channel 20 is provided for insertion of a reference electrode 21. Due to the low dimensional tolerances of all parts involved, the opening 14 for immobilization of the cell 2 is self-aligned with the sensing surface 15 upon assembly of the device 1. The cell chamber is provided with immobilization means 43 for applying a pressure gradient from the cell chamber 11 through the opening 14 towards the perfusion channel 12 by means of air overpressure in the cell chamber 11.

The sensing element 13 is driven with a source-drain follower circuit that fixes the source-drain voltage and drain current to constant values. The reference electrode 21 was connected to the circuit's signal ground which defines the reference potential, against which the output signal V_{GS} is measured at the source connection. In this configuration, changes in V_{GS} are directly proportional to changes in the Nernst potential at the insulator surface. The technical implementation of the drain-source follower is realized using constant current drivers. Acquisition of the gate-source voltage is achieved using a high-resolution data acquisition and control (DAQ) unit (LabJack U6-Pro, LabJack Corp./USA). The DAQ unit can also serve as a controller for an air pressure system used for cell immobilization and solution exchange in the microperfusion system.

In order to monitor transport activities across the cell membrane 3 of a cell 2 the cell 2 is immobilized and aligned with the active area or sensing surface 15 of the field-effect sensor 13. This is achieved by using the two-layer microdevice that is mounted above the sensor surface 1. It comprises the perfusion channel 12 with microfluidic channels in the bottom layer, and a cell chamber 11 with buffer reservoir in the top layer.

The cell chamber 11 serves as an immobilization site and can be funnel-shaped structure with the opening 14 in its center. The cell 2 is pipetted into the funnel where it drops into the opening 14 by the force of gravity. As a result, a part of the cell membrane 3 area is exposed to the liquid perfusion pathway in the perfusion channel 12 below. Proper contact of the cell membrane 3 with the sensing surface 15 is ensured by applying a slight overpressure to the cell chamber 11, pushing the cell 2 further down the opening 14. This also improves the liquid seal between the perfusion channel 12 and the cell chamber 11. After stabilization of the signal at constant perfusion of the exposed cell membrane 3 with buffer solution, the experiment is ready for perfusion with either activating or deactivating solutions. Since the sensor's operating point is kept constant the output signal can be continuously acquired in real-time.

A possibility for applying the slight overpressure in the cell chamber is described in Schaffhauser et al. (Schaffhauser DF et al., Microfluidic platform for electrophysiological studies on Xenopus laevis oocytes under varying gravity levels, 2011, Lab Chip, 11(20):3471-8) which is hereby incorporated by reference.

Fig. 1(b) shows a micrograph of the sensing surface 15 between the source 16 and the drain 17 of the sensing element 13 as seen through the opening 14 (ca. 700 µm in diameter) of the cell chamber 11. The arrow indicates the flow of the fluid through the perfusion channel 12. While performing measurements a cell membrane of an oocyte due to its deformability completely covers the sensing surface 15 (active area) of the sensing element 13.

The microfluidic device 1 for monitoring proton-dependent membrane transport according to the invention is based on the lateral diffusion model. Fig. 2 shows the lateral diffusion model in a schematic close-up of the sensing surface of the device of Fig. 1 covered by a cell membrane.

With the device 1 a proton-selective diffusion barrier 30 can be established that is created by covering the sensing surface 15 of the sensing element 13 with part of the cell membrane 3, respectively part of the vitelline layer 3a of a *Xenopus* oocyte. The vitelline layer 3a of the (defolliculated) oocyte comprises a network of fibrous filaments that surround the cytoplasmic membrane, which itself is not smooth due to numerous microvilli protrusions (not shown). The total thickness of the vitelline layer is estimated to be 2-4. micrometer for stage VI oocytes. We assume the diffusion barrier is narrow compared with the sensor area so that "edge effects" can be neglected and transporters within the sensor region do not "see" the substrate S.

Due to the much higher lateral diffusion rate of the protons compared to other solutes, the detected potential change at the sensing surface 15 is exclusively proton-dependent. Once equilibrated, the proton concentration at the detection site [H+]_{D} (on the sensing surface 15) is equal to the proton concentration at the membrane surface [H+]_{S}, assuming ideal coupling between the sensing surface 15 and the cell membrane surface (including the vitelline layer). The surface potential of the sensing element 13 then reflects the surface pH for steady state membrane transport. In contrast to protons, substrate molecules S that interact with transport proteins 31 at the cell membrane 3 exposed to the bulk solution, will diffuse poorly across the diffusion barrier 30 for two reasons. First, their lateral mobility relative to the protons is greatly reduced and second, they are effectively removed from the local medium by the transport proteins 31 themselves before reaching the sensing surface 15. As a consequence, modulation of substrate concentration [S] in proximity to the sensing surface 15 will be minimal for large migration distances, as present in the device of the invention.

In the following experimental data measured with the microfluidic device according to the invention are given. Experiments were conducted on oocytes overexpressing the electrogenic sodium/phosphate cotransporters PiT-2 (SLC20A2) and NaPi-IIb (SLC34A2), the electroneutral isoform NaPi-IIc (SLC34A3) and the proton/amino acid cotransporter PAT1 (SLC36A1), to demonstrate that the microfluidic device according to the invention allows for non-invasive assays of transport processes, which result in local pH changes under physiological conditions.

### Solutions and reagents:

For storage of oocytes, modified Barth's solution contained (in mM): 88 NaCl, 1 KCI, 0.41 CaCl₂, 0.82 MgSO₄, 2.5 NaHCO₃, 2 Ca(NO₃)₂, 7.5 HEPES, pH was adjusted to 7.5 with Tris and supplemented with 5 mg/l doxycycline and 5 mg/l gentamicine. Standard buffered saline solutions (100 Na) contained (in mM): 100 NaCl, 2 KCI, 1 MgCl₂, 1.8 CaCl₂, 10 HEPES, adjusted to pH 7.5, pH 7.4 or pH 6.5 with TRIS. For test solutions containing activating or inhibiting substances the following stock solutions were used: 1 M (in H₂O) inorganic phosphate (Pi) at pH 7.4, 100 mM (in DMSO) amiloride, 100 mM (in H₂O) L-Proline, 100 mM (in H₂O) γ-Aminobutyric acid (GABA) and 100 mM (in H₂O) sodium phosphonoformate tribasic hexahydrate (PFA). After addition of the reagents, all solutions were filtered using a 0.22 µm filter, prior to filling the solution vials. All standard reagents were purchased from either Sigma-Aldrich or Fluka.

### Oocyte preparation:

Female *Xenopus laevis* frogs were purchased from XenopusExpress (France) or African Xenopus Facility (R. South Africa). The frogs were anesthetized in MS222 (tricainemethanesulphonate) after which portions of ovaries were surgically removed and cut in small pieces. Collagenase treatment on oocytes was done for 45 min with 1 mg/ml of crude type 1A in 100 Na solution (without Ca²⁺) in presence of 0.1 mg/ml trypsin inhibitor type III-O. Healthy stage V-VI oocytes were selected, maintained in modified Barth's solution at 16 °C and injected with typically 5 ng total of cRNA of the α,β,γγ sub-units of the *Xenopus* isoform of ENaC, GAT1, flounder NaPi-IIb, mouse NaPi-IIc, or mouse PAT1 according to procedures previously conducted in our laboratory. After injection, the oocytes were incubated for 3 days at 16 °C in Barth's solution. Non-injected oocytes of the same batch were used as negative controls. All animal handling procedures were approved by Swiss Cantonal and Federal veterinary authorities.

### Procedure for oocyte experiments:

Before the experiment, the oocytes were thoroughly washed in 100 Na solution at pH 7.4 to prevent the diffusion of protons between the cell chamber 11 and the microperfusion channel 12. After priming of the system with 100 Na pH 7.4 solution, the cell was pipetted into the cell chamber 2. The air port plug was then inserted into the cell chamber 2 and a constant air pressure of 5 mbar was applied. The perfusion system was then pressurized at 100 mbar which, in combination with the inlet capillaries (30 cm length, 200 µm inner diameter), resulted in a flow rate of approx. 1 µl/s when one of the solenoid valves was open. The oocyte was perfused with 100 Na solution at pH6.5 (for PAT1) or pH7.4 (all others) for 5 min to create stable steady-state conditions before initiating a transport activation protocol. The perfusion sequence was programmed beforehand. This feature provided high timing accuracy of the solution switching and guaranteed the repeatability of the perfusion sequence.

### pH-sensing characterization of the sensor element:

To determine the response of the sensing element 13 to changes in bulk proton concentration and correlate the changes in output voltage to the pH change, the sensing element 13 was superfused with buffered solutions at varying pH. A slope of -58 mV/pH was found using a three-point extrapolation with standard phosphate buffer solutions at pH4.01, pH7.00 and pH9.21 (Fig. 3(a) and 3(b)). This value was close to the prediction from the Nernst equation (-59 mV/pH), which demonstrated the excellent proton buffering capacity of Ta₂O₅. The sensor-to-sensor variation was insignificant, which resulted from the large sensor structure and precise CMOS manufacturing processes. In a second experiment, the sensing element 13 was superfused with 100 Na solutions at pH 7.50 and pH 7.40 (Fig. 3(c)). The experiment showed an expected decrease of 5.4 mV (i.e. · ΔpH = -0.1) with a rapid transition from the first steady-state signal to the next one. Even though the flow inside the microperfusion channel was laminar, the signal response was not perfect due to cross-diffusion at the junction where the inlet channels meet. Also, there was some dead time between the switching of the valves and the signal response onset due to the relatively large solution exchange volume (approx. 4µl) in relation to the flow rate (1 or 2 µl/s). In total, it took approximately 8 s at 100 mbar (P1) and approximately 5 s at 200 mbar (P2) to reach steady-state conditions after valve actuation (Fig. 3(d)). Nevertheless, for the uptake experiments on oocytes these values are fully acceptable due to the comparatively slow proton diffusion between the membrane surface ([H+]_{S})and the sensor surface ([H+]_{D}).

### Transport experiments on oocytes heterologously expressing various membrane transport proteins:

Fig. 4 shows experiments conducted on oocytes heterologously expressing various membrane transport proteins indicated with their respective controls on non-injected (NI) oocytes showing sensor readout (V_{GS}) as a function of time. Only part of the initial stabilizing baseline region that preceded substrate application is shown: (a) PAT1, (b) NaPillb, (c) NaPi-IIc, (d) PiT-2, (e) Proline control, (f) Pi control, (g) GAT1, (h) ENaC. In each case either the same or representative oocytes from the same batch were pretested using a two-electrode voltage clamp to confirm functional expression. The bars indicate the duration of application of the respective activating and blocking agents. Arrows indicate flux direction of substrate according to the assumed driving force conditions.

### Proline transport mediated by PAT1:

Assays conducted on oocytes heterologously expressing the proton-driven amino acid transporter (PAT1) showed a significant decrease of V_{GS} upon exposure of the cell to a 1 mM proline solution (Fig. 4(a)). The signal reached a steady state after 5 min and returned to the initial baseline after washout of proline. When switching back to the proline-free buffer solution, the signal returned to the initial baseline. PAT1 is known to reversibly bind amino acids and cotransport them stoichiometrically with one proton/amino acid per cycle. Even though PAT1 works bidirectionally, the exposure time of 5 min was apparently not sufficient to significantly change the proton gradient across the cell membrane. The downward deflection of V_{GS} upon exposure to proline translates into an increase in surface pH. This is to be expected since the translocation of free protons by PAT1 results in their depletion at the extracellular membrane surface and a consequent lateral movement of protons away from the sensor region.

### Phosphate transport mediated by NaPi-IIb and PiT-2:

In contrast to PAT1, for oocytes heterologously expressing the electrogenic sodium-coupled phosphate cotransporter (NaPi-IIb) the signal deflection was reversed, which indicated that a decrease in surface pH occurred (Fig. 4(b)). This can be understood when we consider that Pi is present at the membrane surface as both divalent (HPO₄²⁻) and monovalent (H₂PO₄⁻) phosphate species with an assumed pKa = 6.8 under physiological conditions and distributed according to the following equilibrium, HPO₄²⁻ + H⁺ <--> H₂PO₄⁻.

The electrogenic NaPi-IIb translocates 3 Na+ together with one divalent Pi per transport cycle, which results in the increase of [H+]ₛ due to the depletion of the divalent species.

For the electroneutral isoform NaPi-IIc, a detectable pH decrease at the membrane surface is also induced upon exposure to Pi (Fig. 4(c)).This was expected given that NaPi-IIc also prefers divalent Pi, and translocates two Na+ per cycle with no net charge movement. This result also establishes that the sensor (i) is capable of sensing electroneutral transport processes and (ii) does not simply respond to a change in the cell membrane potential as might be predicted for the electrogenic transporters that mediate net charge translocation.

In contrast to the SLC34 family of sodium coupled Pi cotransporters, members of the SLC20 family prefer monovalent Pi (H₂PO₄⁻), and translocate two Na+ per transport cycle together with one positive net charge. The resulting shift of the equilibrium to the right (see equation above) would therefore result in a decrease in [H+]ₛ, which explains the downward deflection (increase in pH) of V_{GS} in PiT-2 upon exposure to Pi (Fig. 4(d)).The amplitude of the change in V_{GS} at steady state was significantly lower compared to NaPi-IIb and NaPi-IIc and most likely resulted from the lower surface expression of protein compared to NaPi-IIb,c. The changes in surface pH registered by the ISFET sensor for NaPi-IIb and PiT-2 were in qualitative agreement with TEVC measurements conducted in combination with surface pH measurements using a pH-sensitive glass microelectrode.

### Control experiments confirm the specificity of the sensor for detecting local ΔpH:

Control experiments on non-injected oocytes showed minimal deflections of V_{GS}, both for Pi and proline (Fig. 4(e),(f)). These may be due to endogenous membrane proteins that could interact with proline and Pi. In particular, the presence of amino acid-modulated membrane activity has been determined in uptake studies. Nonetheless, considering that relatively high concentrations of proline and Pi were used in the experiments, the contribution of the endogenous activity can be neglected when the level of exogenous expression is sufficiently high. To further demonstrate that modulation of proton-independent transport proteins did not induce a potential change at the sensor surface, experiments were conducted on oocytes overexpressing the γγ-aminobutyric acid (GABA) transporter (GAT1) and the epithelial sodium channel (ENaC). GAT1 is a sodium-chloride-dependent cotransporter highly specific for GABA. ENaC is a sodium channel whose conductance can be blocked using amiloride (Kᵢ<1µM). For oocytes expressing either of these membrane proteins, a significant change in V_{GS} could not be resolved. This finding allowed for excluding the presence of detection artifacts at the sensor surface and confirmed the specificity of the sensor to changes in local proton concentration.

### Correlating the ISFET response to transport activity:

Fig. 5 shows the correlation of sensor response with transport activity. Oocytes overexpressing PAT1 were used to correlate the change in membrane surface pH with PAT1 activity. For the oocyte in the sensor configuration, application of proline will result in a net intracellular translocation of protons into the oocyte, which will result in membrane depolarization. The new steady-state membrane potential will be reached that is a function of both the PAT1 expression level as well as the endogenous leak conductance of the oocyte, which can vary from cell to cell. Moreover, the proline transport rate will itself be a function of membrane potential due to voltage-dependent partial reactions in the transport cycle. Therefore, to correlate transport activity with ΔpH on individual oocytes, first the steady-state membrane potential reached during transport under the same conditions as for the sensor assay was determined (inset, Fig 5(b)). For that case, the net membrane current is zero and comprises the inward transporter-related flux that is balanced by an equal, but opposite current mediated by endogenous channels and pumps. The oocyte were then voltage clamped to determine the transporter-related current by subtracting the current in the absence of proline from that obtained with proline to eliminate proline-independent endogenous currents. From this I-V data we could estimate the transport flux corresponding to the steady-state potential for the ISFET assay (Fig. 5(b)). To ensure minimal substrate accumulation, we first tested the response using the TEVC to avoid long substrate exposure incurred with the ISFET system.

A total of six oocytes selected from the same batch were used for comparison. Variance in substrate-dependent current and thus, expression level was within the expected range. A clear correlation between changes in V_{GS} and the substrate-dependent current has been observed (Fig. 5c). This behavior supports the assumption that the change in surface proton concentration is directly proportional to the total substrate current, which for a given turnover rate and driving force, is proportional to the number of active transporters in the membrane. Indeed, this is to be expected if the dependence of protons on the translocation of a substrate-specific substance is stoichiometric. The substrate turnover rate is then proportional to [H+]ₛ, which allows quantitative substrate activation and inhibition studies. This implies that a high coupling strength between [H+]ₛ and [H+]_{D} is maintained to minimize secondary effects compromising the linearity of concentration change. It is assumed that optimizing the geometry of the structures involved in the immobilization of oocytes (hole diameter, sensor-to-hole distance) will lead to improved coupling strength between the membrane transport proteins and the sensor surface, without compromising the effectiveness of the proton-selective diffusion barrier.

### LIST OF REFERENCE NUMBERS

- 1: microfluidic device
- 2: cell (oocyte)
- 3: cell membrane
- 11: cell chamber
- 12: perfusion channel
- 13: sensing element
- 14: opening
- 15: sensing surface
- 16: source
- 17: drain
- 18: gate
- 19: inlet channel
- 20: outlet channel
- 21: reference electrode
- 22: gate insulator material
- 30: proton-selective diffusion barrier
- 31: transporter protein
- 40: support
- 41: rectangular piece
- 42: block
- 43: immobilization means

## Claims

1. Microfluidic device (1) for monitoring transport activity across a cell membrane (3, 3a) of a cell (2), the device (1) comprising a cell chamber (11) and a perfusion channel (12), wherein the cell chamber (11) is connected with the perfusion channel (12) through an opening (14), **characterized in that** the microfluidic device (1) further comprises a sensing element (13) having a sensing surface (15), wherein the sensing surface (15) is arranged in the perfusion channel (12) and aligned with the opening (14) such that the sensing surface (15) is completely coverable by a cell membrane (3, 3a) of a cell (2) placed in the cell chamber (11).

2. Microfluidic device (1) according to claim 1, wherein the sensing element (13) is a field-effect transistor with a source (16), a drain (17) and a gate (18), preferably an ion-sensitive field-effect transistor, and wherein a gate insulator material(22) forms the sensing surface (15).

3. Microfluidic device according to one of the preceding claims, wherein the sensing surface (15) is formed by a pH-sensitive layer preferably of Si₃N₄, Al₂O₃ or Ta₂O₅.

4. Microfluidic device according to one of the preceding claims, wherein the microfluidic device comprises immobilization means (43) to immobilize the cell (2) on the sensing surface (13) to establish a proton-selective diffusion barrier (30) between the sensing surface (15) and the perfusion channel (12).

5. Microfluidic device according to claim 4, wherein the immobilization means (43) are means for applying a pressure gradient from the cell chamber (11) through the opening (14) towards the perfusion channel (12).

6. Microfluidic device according to claim 4 or 5, wherein the immobilization means (43) are means for applying air overpressure in the cell chamber (11).

7. Microfluidic device according to claim 4 or 5, wherein the immobilization means (43) are means for applying underpressure at an outlet of the perfusion channel (12).

8. Microfluidic device according to one of the preceding claims, wherein the perfusion channel (12) comprises at least one inlet channel (19) and one outlet channel (20).

9. Microfluidic device according to one of the preceding claims, wherein a reference electrode (21) is placed in the perfusion channel (12), preferably in the region of an outlet channel (20) of the perfusion channel (12).

10. Multichannel microfluidic device for monitoring transport activity across a cell membrane (3, 3a) of a cell (2) comprising several microfluidic devices (1) according to one of claims 1 to 9 arranged in an array.

11. Method for monitoring transport activity across a cell membrane (3, 3a) of a cell (2) using a microfluidic device (1) according to one of claims 1 to 9 or a multichannel microfluidic device according to claim 10 comprising the following steps:
(a) providing a cell (2) in the cell chamber (11);
(b) establishing a proton-selective diffusion barrier (30) between the sensing surface (15) and the perfusion channel (12);
(c) monitoring a change in proton concentration at the sensing surface ([H+]_{D}) due to transport activitiy across the cell membrane (3, 3a) of the cell (2).

12. Method according to claim 11, wherein the proton-selective diffusion barrier (30) is established by immobilizing the cell (2) on the sensing surface (15) by applying a pressure gradient from the cell chamber (11) towards the perfusion channel (12).

13. Method according to one of claims 11 or 12, wherein the pressure gradient force is applied by applying air pressure in the cell chamber (11), to press the cell (2) into the opening (14) and onto the sensing surface (15).

14. Method according to one of claims 11 or 12, wherein the pressure gradient is applied by applying a negative pressure at the outlet of the perfusion channel (12) to suck the cell (2) into the opening (14) and onto the sensing surface (15).
